# EUROPÄISCHE PATENTSCHRIFT

(11) **EP 2 590 527 B1**
(45) Veröffentlichungstag und Bekanntmachung des Hinweises auf die Patenterteilung: **01.10.2014**
(21) Anmeldenummer: 11730917.9
(22) Anmeldetag: 08.07.2011
(51) Int. Cl.: A43B 7/22, A43B 23/22, A43B 7/14

(54) **Stützspange für Schuheinlagen**
Supporting brace for footwear inserts
Croisillon de soutien pour semelles orthopédiques

(30) Priorität: 09.07.2010 DE 102010027418
(43) Veröffentlichungstag der Anmeldung: 15.05.2013
(73) Patentinhaber: Bauerfeind AG, 07937 Zeulenroda-Triebes (DE)
(72) Erfinder: JAEGER, Thorsten, 08527 Plauen (DE); ANDERSCH, Christian, 07937 Zeulenroda-Triebes (DE); PANZER, Dominique, 07937 Zeulenroda-Triebes (DE); GLINDEMANN, Olaf, 99094 Erfurt (DE)
(74) Vertreter: Wohlfahrt, Jan Günther
(86) Internationale Anmeldenummer: PCT/EP2011/003410
(87) Internationale Veröffentlichungsnummer: WO 2012/003993

(56) Entgegenhaltungen:
- WO-A1-2008/136559
- US-A- 1 399 447
- US-A- 1 907 995
- US-A- 2 498 624

## Beschreibung

Die Erfindung betrifft eine orthopädische Schuheinlage zur Stützung und Führung des Fußes und stellt neuartige Einlagenkerne für Schuheinlagen bereit, die eine sich im Wesentlichen in Längsrichtung des Einlagenkerns erstreckende Stützspange mit gegeneinander verschränkten diagonal verlaufenden Stützarmen aufweist.

Orthopädische Schuheinlagen werden bekanntermaßen in Schuhwerk eingelegt und wirken dort als kraftausgleichend oder - verteilend, um den Fuß, besonders das Fußgewölbe, zu stützen und in seinem natürlichen Bewegungsablauf zu führen. Dadurch kann die Statik des gesamten Körpers "von der Basis her" verbessert werden, indem die Einlage die Fußgelenke, und in Folge auch Knie- und Hüftgelenke, in die physiologisch korrekte Position zurückführt oder dort hält. Schuheinlagen können deshalb nicht nur unmittelbar zur Linderung akuter Fußbeschwerden, sondern auch zur Prophylaxe und Therapie von Beschwerden und Erkrankungen des Kniegelenks, der Hüfte, des Beckens sowie der Wirbelsäule eingesetzt werden. Indikationen für orthopädische Schuheinlagen sind besonders Knie- und Hüftfehlstellungen, Senk-Spreizfuß, Knick-Senkfuß, Plattfuß und Hohlfuß.

In der Regel sind Schuheinlagen als "langsohlige" Varianten ausgeführt, die die gesamte Innensohle, und damit Ferse, Ballen und Zehen des Fußes, in einem Schuh überdecken. Andere Varianten sind kürzere, so genannte Dreivierteleinlagen, die von der Ferse bis zum Zehenballen reichen. Die Positionierung der Schuheinlage am Fuß erfolgt in der Regel über die Ferse. Dort kann eine Aussparung, Materialverdünnung oder ein nachgiebiges Polsterelement vorgesehen sein, worüber der Fersenabschnitt der Einlage an dem Fersenballen zentriert wird.

Bekannte orthopädische Schuheinlagen sind aus mehreren Materiallagen mehrschichtig aufgebaut. In der Regel sind zumindest eine polsternde Bezugs- oder Deckschicht auf der zum Fuß gewandten Oberseite sowie eine mechanisch stabile, formgebende, stützende und gegebenenfalls polsternde Schicht auf der zum Schuh gewandten Unterseite vorgesehen. Um der Einlage die für deren Funktion erforderliche stützende und führende Funktion zu verleihen, kann zusätzlich ein mechanisch festes Versteifungselement vorgesehen sein, das bekanntermaßen bei der Herstellung in oder zwischen die Materiallagen eingelegt und mit diesen mechanisch verbunden wird. Die Stützwirkung solcher Versteifungselemente oder "Einlagenkerne" ist in bekannten orthopädischen Einlagen aber häufig unzureichend, vor allem dann, wenn die Einlage nicht individuell an das Gewicht des Patienten oder beispielsweise die Therapie angepasst werden kann. Gewünscht ist auch eine Führungsfunktion, die das Abrollverhalten des Fußes steuert und eine möglichst physiologische Ganglinie ermöglicht. Bekannte Einlagen können diese Führungsfunktion teilweise nur sehr unzureichend erfüllen. Bekannte orthopädische Einlagen tragen außerdem stark auf, um die für die Funktion erforderliche mechanische Stabilität zu erreichen, und erfordern dann meist speziell an die Einlagen angepasstes Schuhwerk, das noch genügend Raum über dem Fußbett lässt. Die Einsatzmöglichkeit für bekannte orthopädische Schuheinlagen in normalen Schuhen ist daher begrenzt.

Die Dokumente US1907995 und US2498624 offenbaren ein Einlagenkern für eine Schuheinlage, enthaltend einen Träger und eine darin angeordnete Stützspange mit hinteren Stützarmen und vorderen Stützarmen, die sich im Bereich des Fußgewölbes kreuzen und zu gegenüberliegenden Enden des Einlagenkerns hin auslaufen mit zur Ferse hin gerichteten hinteren Abschnitten und zur Fußspitze hin gerichteten vorderen Abschnitten, wovon die jeweils zur Fußinnenseite hin gerichteten Abschnitte innere Abschnitte und die jeweils zur Fußaußenseite hin gerichteten Abschnitte äußere Abschnitte bilden.

Die Erfindung hat sich die Aufgabe gestellt, orthopädische Schuheinlagen weiterzuentwickeln, um die vorgenannten Nachteile zu überwinden. Besonders soll erreicht werden, dass bei einem im Wesentlichen gleichartigen Aufbau der Einlagen, besonders bei der Herstellung, eine leichte Anpassbarkeit in Bezug auf Stützfunktion und/oder Führungsfunktion an den Patienten gegeben ist. Im Vordergrund steht hier auch die Bereitstellung einer neuartigen Konstruktion, die eine vereinfachte Produktion einer Reihe von Einlagen mit unterschiedlich stark ausgeprägter mechanischer Stütz- oder Führungsfunktion in ein und demselben automatisierten Herstellungsprozess unter Verringerung der Rüstzeiten ermöglicht.

Das technische Problem wird vollständig gelöst durch die Bereitstellung eines stützenden Einlagenkerns für eine Schuheinlage zur Stützung oder Führung eines Fußes, wobei der Einlagenkern einen Träger und eine darin angeordnete Stützspange, besonders aus mechanisch festerem Material, die die mechanische Stützfunktion oder Führungsfunktion der gesamten Einlage vermitteln kann, enthält. Die Stützspange weist erfindungsgemäß sich insbesondere im Bereich des Fußgewölbes kreuzende, das heißt gegeneinander verschränkte, im Wesentlichen diagonal verlaufende Stützarme, die zu gegenüberliegenden Enden des Einlagenkerns hin auslaufen, auf. Es sind zumindest zwei hintere Stützarme und zumindest zwei vordere Stützarme vorgesehen, die die Stützspange des Einlagenkerns bilden. Erfindungsgemäß erstrecken sich die Stützarme jeweils diagonal in dem Einlagenkern und bevorzugt im Wesentlichen entlang dessen Längsausdehnung und damit der Schuheinlage. Es bilden dabei jeweils zwei sich im Wesentlichen diagonal gegenüberliegenden Stützarme eine Stützbrücke als tragendes Element der Stützspange. Eine erste Stützbrücke wird erfindungsgemäß von einer durch andere sich ebenfalls im Wesentlichen diagonal gegenüberliegende Stützarme gebildeten weiteren Stützbrücke querend gekreuzt. Die Stützspange ist also bevorzugt so in dem Einlagenkern angeordnet, dass sie sich in ihrer Längsausdehnung im Wesentlichen entlang der Längsausdehnung des Einlagenkerns oder bevorzugt der Einlage selbst erstreckt. Damit erstrecken sich die gekreuzten Stützarme beziehungsweise Stützbrücken vorzugsweise im Wesentlichen von der Ferse oder von dem Übergang zwischen Fußgewölbe und Ferse bis hin zum Zehenballen.

Die Erfindung sieht also vor, innerhalb eines Einlagenkerns aus einem Träger eine im Wesentlichen aus zumindest oder genau zwei gekreuzten Stützbrücken, die gegeneinander verschränkt diagonal in dem Einlagenkern verlaufen, aufgebaute Stützspange anzuordnen. Es ist dabei vorgesehen, dass diese Stützspange im Wesentlichen die mechanische Stütz- und Führungsfunktion des Einlagenkerns und damit der gesamten Schuheinlage, deren Bestandteil der Einlagenkern ist, übernimmt.

Vorteilhafterweise kann diese Einlage dünner ausgeführt werden als bekannte Schuheinlagen mit vergleichbarer mechanischer Stütz- oder Führungsfunktion. Während bekannte Schuheinlagen die Stütz- oder Führungsfunktion besonders über eine große Stärke eines Plattenmaterials eines elastischen Schaumwerkstoffs erreichen und deshalb an den besonders zu stützenden oder führenden Stellen der Einlage dick sein müssen, sodass die Einlage aufträgt und in bestimmte schmale Schuhe nicht mehr passen würde, kann gemäß der Lehre der vorliegenden Erfindung auf dickes, mechanisch festes Plattenmaterial verzichtet werden, um die mechanische Stütz- oder Führungsfunktion zu erzielen.

Die Ausprägung der mechanischen Stütz- oder Führungsfunktion eines Stützarms der Stützspange ist in einer ersten Ausgestaltung des erfindungsgemäßen Einlagenkerns in einem höheren Festigkeitsmodul des Werkstoffs des Stützarms begründet. Alternativ oder zusätzlich ist die Ausprägung in einer größeren Materialdicke des Einlagenkerns im Bereich des Stützarms begründet. In einer alternativen oder zusätzlichen Variante ist die Ausprägung in einer größeren Materialbreite im Bereich des Stützarms begründet.

Die Erfindung sieht vor, dass die Stützspange in ihrer konkreten Formgestaltung und Ausprägung innerhalb des Einlagenkerns zum Erreichen einer bestimmten Stütz- oder Führungsfunktion einfach variabel gestaltbar ist, ohne dass sich dadurch die Gesamtstruktur des Einlagenkerns und damit der gesamten Einlage, besonders in Bezug auf die äußeren Abmessungen, besonders der Dicke der Einlage, verändert. Vorteilhafterweise können die übrigen in der Einlage verwendeten Strukturen und Werkstoffe unverändert bleiben. Die Erfindung erlaubt somit besonders ein und denselben Herstellungsprozess für einen Einlagenkern beziehungsweise für eine Schuheinlage, worin in Bezug auf die Stütz- oder Führungsfunktion der Einlage unterschiedlich ausgeprägte Einlagenkerne ohne Weiteres vollautomatisch herstellbar sind.

Die unterschiedliche Ausprägung der Stütz- oder Führungsfunktion wird gemäß der Erfindung besonders dadurch erreicht, dass die Stützspange einzelne individuell ausgestaltbare Stützarme aufweist, die durch Änderung der Werkstoffeigenschaften und/oder besonders durch Anpassung/Änderung der äußeren Form (Dicke, Breite) in ihrer mechanischen Festigkeit individuell herstellbar sind. Beispielsweise kann in einer zweiten Produktionscharge des Einlagenkerns oder der Einlage mindestens ein Stützarm in einer anderen Materialstärke und/oder besonders in einer anderen Breite ausgeführt sein als in einer ersten Charge. Ein breiterer und/oder dickerer Stützarm führt, besonders lokal, zu einer höheren mechanischen Festigkeit und damit zu einer anders ausgeprägten Stütz- oder Führungsfunktion der Einlage. Soll umgekehrt eine zweckmäßig verminderte Stützwirkung und damit eine höhere mechanische Nachgiebigkeit in einer bestimmten Zone der Einlage erreicht werden, ist beispielsweise vorgesehen, vor allem den dieser Zone/Abschnitt zugeordneten entsprechenden Stützarm mit geringerer Materialstärke, besonders in geringerer Breite auszuführen.

Die Stützspange ist innerhalb des Trägers aus einen im Vergleich zu den übrigen Werkstoffen des Einlagenkerns, besonders also des Trägers, und auch besonders im Vergleich zu den übrigen Materiallagen der Schuheinlage mechanisch steifer, das heißt widerstandsfähiger ausgeprägt und weist einen höheren Elastizitätsmodul auf. Dazu ist in einer ersten Ausgestaltung der Erfindung vorgesehen, dass die Stützspange aus einem steiferen Werkstoff als die übrigen Elemente des Einlagenkerns ausgeführt ist.

In der alternativen oder zusätzlichen Ausgestaltung ist die Stützspange integraler Bestandteil des Trägers und einstückig mit dem Träger und aus demselben Werkstoff ausgebildet. Die Stützspange grenzt sich von dem Träger besonders durch eine höhere Materialstärke ab. Das heißt, in diesem Fall wird die mechanisch steifere Stützspange durch eine Erhöhung der Materialstärke in dem die Stützspange bildenden Abschnitt des Trägers erreicht. Soll hier eine individuelle Erhöhung oder Erniedrigung der Steifigkeit einer oder mehrerer Stützarme erreicht werden, ist in dieser Ausgestaltung vorgesehen, die Materialstärke lokal im Bereich dieses Stützarms zu erhöhen beziehungsweise zu erniedrigen und/oder bevorzugt die den Stützarm bildende Materialzone in dem Träger innerhalb einer variablen Zone zu verbreitern beziehungsweise zu verschmälern.

In einer ersten Ausführung ist der Einlagenkern also mehrteilig aus einem Träger und mindestens einer davon separat herstellbaren Stützspange gebildet, die bevorzugt aus einem anderen, insbesondere mechanisch festeren Werkstoff ausgebildet ist. In einer alternativen Ausführung ist der Einlagenkern also einstückig und einteilig ausgebildet, wobei besonders Träger und Stützspange aus demselben Werkstoff und bevorzugt in einem einheitlichen Verfahren zusammen herstellbar sind.

Vorteilhafterweise erlaubt die Erfindung, die einfache Anpassung oder Variation der mechanischen Festigkeit einzelner oder mehrerer Stützarme der Stützspange innerhalb eines automatisierten Herstellprozesses durch minimale Anpassungen des entsprechenden Werkzeugs mit kürzester Rüstzeit durchzuführen. Bevorzugt ist hierbei ein modularer Aufbau von Einlagenkern und/oder Werkzeug. So erlaubt die Erfindung, dass innerhalb ein und desselben Basiswerkzeugs zur Herstellung des Einlagenkerns oder der Stützspange oder alternativ der gesamten Einlage nur einzelne Werkzeugteilelemente ausgewechselt werden müssen, um Stützspangen, Einlagenkerne beziehungsweise komplette Schuheinlagen mit bestimmter mechanischer Stützfunktion zu erhalten. Die automatische Herstellung umfasst auch den automatischen Wechsel des Teilwerkzeugelements zur Festlegung der mechanischen Festigkeit der Stützspange nach den Maßgaben des Steuerprogramms. Die Erfindung ist nicht auf die hier näher beschriebenen Verfahrensprinzipien zur automatisierten Herstellung von Einlagenkernen beschränkt. Sie ist beispielsweise sowohl auf an sich bekannte Spritzgießverfahren wie injection molding, als auch auf an sich bekannte Mehrschichtlaminierverfahren anwendbar.

In einer bevorzugten Ausgestaltung ist die formfeste Stützspange ein Spritzgussteil. Bevorzugt sind thermoplastische, insbesondere schlagzähe Materialen. Bevorzugt sind Polyethylen, Polypropylen, Polyurethan und insbesondere Ethylenvinylacetat (EVA) sowie Mischungen aus diesen Komponenten.

In Bezug auf die mechanische Stützfunktion individuell angepasste Einlagenkerne und Einlagen sind so höchst wirtschaftlich herstellbar. Gleichzeitig wird durch die einfache Automatisierbarkeit des Herstellungsprozesses die von ansonsten manuell durchgeführten individuellen Herstell- oder Anpassungsverfahren bekannte Bauteilstreuung in Bezug auf die erzielten Ergebnisse und die Qualität des hergestellten Produktes vermieden. Die Erfindung erlaubt erstmalig die automatisierte industrielle Herstellbarkeit einer Vielzahl individuell konfektionierter orthopädischer Schuheinlagen in hoher gleichbleibender Qualität und hoher Wirtschaftlichkeit. Ähnliche Verfahren sind aus dem Stand der Technik nicht bekannt.

Es ist im Detail vorgesehen, dass der erfindungsgemäße Einlagenkern in hintere Abschnitte, die, innerhalb der Schuheinlage, zur Ferse hin gerichtet sind, und in vordere Abschnitte, die zur Fußspitze hin gerichtet sind, eingeteilt werden kann. Weiter ist der Einlagenkern in zur Fußinnenseite gerichtete innere Abschnitte und in zur Fußauβenseite gerichtete äußere Abschnitte einteilbar. Insgesamt ergibt sich eine Einteilung in vier Quadranten aus einem hinteren inneren Abschnitt, einem hinteren äußeren Abschnitt, einem vorderen inneren Abschnitt und einem vorderen äußeren Abschnitt. Die Erfindung sieht besonders vor, dass zumindest ein in einem vorderen oder in einem hinteren inneren Abschnitt verlaufender Stützarm der Stützspange von zumindest einem in demselben vorderen beziehungsweise hinteren äußeren Abschnitt verlaufenden gegenüberliegenden Stützarm in Bezug auf dessen mechanische Stützfunktion unterschiedlich ausgeprägt ist. Beispielsweise weist demnach der hintere Stützarm, der im inneren Abschnitt verläuft, eine andere mechanische Eigenschaft als der andere hintere Stützarm, der im äußeren Abschnitt verläuft, auf. Ebenso kann dies für die beiden vorderen Stützarme vorgesehen sein, die sich in derselben Weise in ihrer mechanischen Stabilität unterscheiden können.

In einer Ausgestaltung sind im Detail die sich jeweils diagonal gegenüberliegenden vorderen und hinteren Stützarme, die gemeinsam eine diagonale Stützbrücke bilden, in der Ausprägung ihrer mechanischen Stabilität und Stützfunktion aneinander angepasst und bevorzugt angeglichen. Es ist in Varianten davon vorgesehen, dass beide entsprechend mechanisch fester oder alternativ jeweils mechanisch weniger fest als die übrigen Stützarme der Stützspange ausgestaltet sind. Demgemäß ist in einer besonderen Variante im Detail vorgesehen, dass der in dem vorderen äußeren Abschnitt erstreckende vordere Stützarm im Vergleich zu dem in dem vorderen inneren Abschnitt erstreckenden vorderen Stützarm in Bezug auf dessen mechanische Stützfunktion stärker ausgeprägt ist. In einer bevorzugt zusätzlichen Ausgestaltung ist der sich in den hinteren äußeren Abschnitt erstreckende hintere Stützarm im Vergleich zu dem sich in den hinteren inneren Abschnitt erstreckende hintere Stützarm in Bezug auf dessen mechanische Stützfunktion schwächer ausgeprägt. Die in den diametral gegenüberliegenden hinteren inneren und vorderen äußeren Abschnitten erstreckenden vorderen und hinteren Stützarme bilden eine Stützbrücke, die im Vergleich zu einer durch die anderen dazu quer erstreckenden Stützarmen gebildete zweite Stützbrücke, welche die erste Stützbrücke quert, in Bezug auf die mechanische Stützfunktion stärker ausgeprägt ist.

Gegenstand der Erfindung ist auch eine orthopädische Schuheinlage, die den erfindungsgemäßen Einlagenkern enthält, sowie ein Verfahren zu deren Herstellung. Das Verfahren ist automatisiert durchführbar: In einem ersten Schritt kann der Einlagenkern aus einem Träger und einer gegebenenfalls separat hergestellten in Bezug auf die mechanische Festigkeit individuell ausgebildeten Stützspange hergestellt werden. In einem zweiten Schritt wird der individualisiert hergestellte Einlagenkern in einer herkömmlichen Endverarbeitung zu einer Schuheinlage ergänzt.

In einer besonderen Ausgestaltung ist die orthopädische Schuheinlage gebildet aus den erfindungsgemäß strukturierten Einlagenkern, zusammen mit einer Grundsohle und gegebenenfalls einer dem Fuß zugewandten Bezugs- oder Deckschicht. In einer ersten Ausgestaltung ist der Einlagenkern in an sich bekannter Weise in die Grundschicht eingelegt und mit dieser mechanisch fest verbunden. In einer besonderen Ausgestaltung wird die Grundschicht durch gezielte Umschäumung des erfindungsgemäßen Einlagenkerns gebildet und mit dem Einlagenkern als integrales Formteil erhalten. Dabei ist beispielsweise vorgesehen, dass der erfindungsgemäße Einlagenkern von Formmasse gezielt vollständig oder abschnittsweise umgossen wird, um die orthopädische Schuheinlage zu bilden. Die Erfindung ist nicht auf diese Art der Herstellung beschränkt. Alternative Verfahren sind die Verklebung des Einlagenkerns mit weiteren Komponenten, um die Schuheinlage zu bilden. Bekannte Verfahren sind hier ein schichtweises Verkleben von Schaummaterial auf der Fuß zugewandten und auf der Fuß abgewandten Seite des Einlagenkern, sodass dieser in die so gebildete Schuheinlage integriert ist. Daneben existieren weitere bekannte Verfahren, um Schuheinlagen zu bilden. Diese können vom Fachmann in Kenntnis der erfindungsgemäßen Lehre so abgewandelt werden, dass der erfindungsgemäße Einlagenkern dort eingesetzt werden kann, um eine Schuheinlage zu bilden. Es wird beispielsweise in einer ersten Ausgestaltung des Herstellverfahrens der Einlagenkern in ein Gießwerkzeug eingelegt, und anschließend werden eine oder mehrere plastische Formmassen in das Gießwerkzeug eingeleitet, besonders eingespritzt, und zwar derart, dass der Einlagenkern von der mindestens einen Formmasse entweder abschnittsweise oder vollständig umflossen und umschlossen wird. Die Formmasse wird in der Gießform an dem Einlagenkern ausgehärtet, um die orthopädische Schuheinlage zu bilden.

In der Schuheinlage kann zusätzlich eine Bezugsschicht vorgesehen sein, um beispielsweise die Hautverträglichkeit und/oder die Haft- oder Gleiteigenschaften zwischen dem Kern der Einlage und dem zu dämpfenden oder zu stützenden Fuß zu verbessern. In einer entsprechenden Ausgestaltung des Herstellverfahrens wird dazu der Einlagenkern zusammen mit der Bezugsschicht unmittelbar mit der Ausbildung der Grundschicht in dem Gießwerkzeug eingeführt und als Bestandteil der Schuheinlage erhalten. Es wird ein integrales Formteil in einem einzigen Herstellprozess erhalten. Das so erhaltene Formteil kann unmittelbar ohne weitere substanzielle Verarbeitungsschritte als orthopädische Schuheinlage eingesetzt werden.

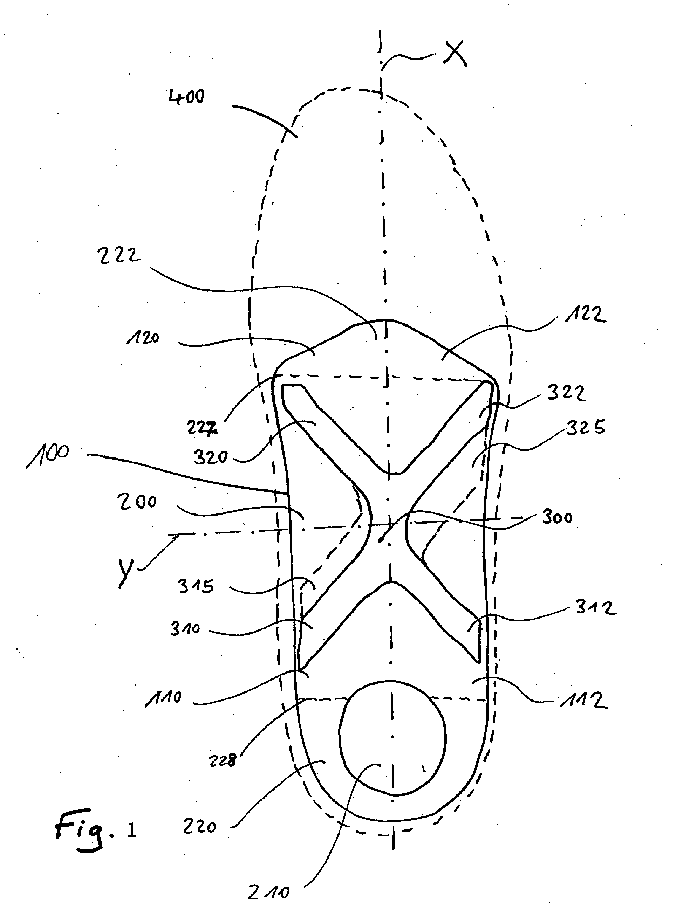

Die Erfindung wird durcn die beigefügte Figur und die Figurenbeschreibung näher illustriert, ohne dass diese beschränkend zu verstehen wäre.

Die Figur zeigt eine Ausführungsform des erfindungsgemäßen Einlagenkerns 100 zum Einsatz in eine orthopädische Einlegesohle 400. Dieser weist einen Träger 200 und eine Stützspange 300 auf. Es skizzieren die Linien X und Y jeweils die Aufteilung des Einlagenkerns in vier Abschnitte: Hinterer innerer Abschnitt 110, hinterer äuβerer Abschnitt 112, vorderer innerer Abschnitt 120 und vorderer äußerer Abschnitt 122. Die Stützspange 300 weist gegeneinander verschränkte Stützarme 310, 320, 312, 322 auf, die sich jeweils in den vorgenannten Abschnitten 110, 120, 112 und 122 erstrecken. Die sich in den jeweils hinteren Abschnitten gegenüberliegenden Stützarmen 310 und 312 sind gemäß der Erfindung mechanisch unterschiedlich fest ausgestaltet, sodass diese eine unterschiedlich starke Stützfunktion ausüben können. In der dargestellten Ausführung tritt dies auch auf die beiden vorderen Stützarme 320 und 322 zu. In der dargestellten Ausführungsform bilden die sich diametral gegenüberliegenden Stützarme 310 und 322, die sich in dem hinteren inneren Abschnitt 110 und dem diesen gegenüberliegenden vorderen äußeren Abschnitt 122 erstrecken, eine mechanisch besonders stabile Stützbrücke, die erfindungsgemäß gegenüber den dazu querend verlaufenden übrigen Stützarmen 312 und 320 mechanisch stabiler ausgeprägt sind und eine stärkere *Stützfunktion ausüben* können. Die Erfindung ist nicht auf diese konkret dargestellte Ausführungsform beschränkt.

Gemäß der Erfindung weist zumindest ein Stützarm, bevorzugt zwei diagonal gegenüberliegende Stützarme, eine individuell konfektionierbare höhere mechanische Festigkeit auf. In der Figur ist dies an den Stützarmen 310 und 322 verwirklicht. Diese weisen dazu jeweils eine variable Zone zur Verwirklichung einer individuell konfektionierbaren Materialverbreiterung 315 und 325 auf. Die Materialverbreiterung ist im Bereich der Zone 315, 325 variabel gestaltet, sodass durch minimale Änderung innerhalb der Zone 315, 325 durch die zur Herstellung der Stützspange 300 verwendeten Mittel eine individuelle Konfektionierung erreicht werden kann.

Zur besseren Positionierung und Formgebung des Einlagenkerns mit der Einlage weist der Einlagenkern optional im Bereich der hinteren Abschnitte eine Aussparung 210 auf, die besonders unterhalb der Ferse des Fußes zu liegen kommt und dort zentriert wird. Weiter weist der Einlagenkern optional Materialverdünnungen 228, 227 auf, die bewegliche Abschnitte 220, 222 zur Anpassung des ansonsten mechanisch steifen Einlagenkerns an die Schuhform ermöglichen. Der Einlagenkern 100 ist ein integraler Bestandteil einer orthopädischen Einlegesohle 400.

## Patentansprüche

1. Einlagenkern (100) für eine Schuheinlage, enthaltend einen Träger (200) und eine darin angeordnete Stützspange (300) mit hinteren Stützarmen (310, 312) und vorderen Stützarmen (320, 322), die sich im Bereich des Fußgewölbes kreuzen und zu gegenüberliegenden Enden des Einlagenkerns hin auslaufen, mit zur Ferse hin gerichteten hinteren Abschnitten (110, 112) und zur Fußspitze hin gerichteten vorderen Abschnitten (120, 122), wovon die jeweils zur Fußinnenseite hin gerichteten Abschnitte innere Abschnitte (110, 120) und die jeweils zur Fußaußenseite hin gerichteten Abschnitte äußere Abschnitte (112, 122) bilden, **dadurch gekennzeichnet, dass** die sich im Wesentlichen in den diametral gegenüberliegenden hinteren inneren (110) und vorderen äußeren (122) Abschnitten erstreckenden Stützarme (310, 322) eine den Einlagenkern (100) querende erste Stützbrücke bilden, die im Vergleich zu den anderen sich dazu quer erstreckenden Stützarmen (312, 320) in Bezug auf die mechanische Stützfunktion stärker ausgeprägt ist.

2. Einlagenkern (100) nach Anspruch 1, wobei die Ausprägung der Stärke der mechanischen Stützfunktion des Stützarms in einem höheren Festigkeitsmodul des Werkstoffs begründet ist.

3. Einlagenkern (100) nach einem der vorstehenden Ansprüche, wobei die Ausprägung der Stärke der mechanischen Stützfunktion des Stützarms in einer größeren Materialdicke des Stützarms begründet ist.

4. Einlagenkern (100) nach einem der vorstehenden Ansprüche, wobei die Ausprägung der Stärke der mechanischen Stützfunktion des Stützarms in einer größeren Materialbreite des Stützarms begründet ist.

5. Einlagenkern (100) nach Anspruch 4, wobei im Bereich des Stützarms eine variable Zone (315, 325) zur Anpassung der Materialbreite vorgesehen ist.

6. Einlagenkern (100) nach einem der vorstehenden Ansprüche, der mehrteilig aus mindestens einem Träger (200) und mindestens einer Stützspange (300) ausgebildet ist.

7. Einlagenkern (100) nach einem der vorstehenden Ansprüche, worin Träger (200) und Stützspange (300) zusammen einteilig ausgebildet sind.

8. Orthopädische Schuheinlage, enthaltend den Einlagenkern (100) nach einem der vorstehenden Ansprüche.

## Claims

1. Insert core (100) for a shoe insert, comprising a carrier (200) and a support clasp (300) arranged therein with rear support arms (310, 312) and frontal support arms (320, 322), which intersect in the area of the arch of the foot and taper towards the opposite ends of the insert core, with rear sections (110, 112) aligned towards the heel and frontal sections (120, 122) aligned towards the tip of the foot; with sections respectively facing the inside of the foot forming internal sections (110, 120) and sections respectively facing the outside of the foot forming external sections (112, 122), **characterized in that** support arms (310, 322) extending substantially in diametrically opposite rear internal sections (110) and frontal external sections (122) forming a first support bridge crossing the insert core (100), which is embodied stronger in reference to the other support arms (312, 322) extending transversally thereto with regards to the mechanic support function.

2. Insert core (100) according to claim 1, wherein the embodiment of the strength of the mechanic support function of the support arm is caused by the higher strength module of the material.

3. Insert core (100) according to one of the previous claims, wherein the embodiment of the strength of the mechanic support function of the support arm is caused by the greater material thickness of the support arm.

4. Insert core (100) according to one of the previous claims, wherein the embodiment of the strength of the mechanic support function of the support arm is caused by a greater material width of the support arm.

5. Insert core (100) according to claim 4, wherein a variable zone (315, 325) is provided in the area of the support arm to adjust the material width.

6. Insert core (100) according to one of the previous claims, which is embodied in several parts comprising at least one carrier (200) and at least one support clasp (300).

7. Insert core (100) according to one of the previous claims, wherein carrier (200) and support clasp (300) are embodied together in one piece.

8. Orthopedic shoe insert comprising the insert core (100) according to one of the previous claims.

## Revendications

1. Noyau de semelle (100) pour une semelle de chaussure, comprenant un support (200) et un croisillon de soutien (300) disposé dans celui-ci et ayant des bras de support postérieurs (310, 312) et des bras de support antérieurs (320, 322) qui se croisent au niveau de la voûte plantaire et se terminent vers extrémités opposées du noyau de semelle, avec des parties postérieures (110, 112) orientées vers le talon et des parties antérieures (120, 122) orientées vers la pointe du pied, dont les parties orientées vers le côté intérieur du pied respectivement forment des parties intérieures (110, 120) et les parties orientées vers le côté extérieur du pied respectivement forment des parties extérieures (112, 122), **caractérisé en ce que** les bras de support (310, 322) qui s'étendent sensiblement dans les parties postérieures et intérieures (110) et les parties antérieures et extérieures (122) diamétralement opposées, forment un premier pont d'appui traversant le noyau de semelle (100) qui est formé de manière plus forte que les autres bras de support (312, 320) s'étendant transversalement à celui-ci en termes de la fonction de support mécanique.

2. Noyau de semelle (100) selon la revendication 1, dans lequel la dimension de force de la fonction de support mécanique du bras de support est fondée sur un module de rigidité élevé du matériau.

3. Noyau de semelle (100) selon l'une quelconque des revendications précédentes, dans lequel la dimension de force de la fonction de support mécanique du bras de support est fondée sur une épaisseur de matériau plus grande du bras de support.

4. Noyau de semelle (100) selon l'une quelconque des revendications précédentes, dans lequel la dimension de force de la fonction de support mécanique du bras de support est fondée sur une largeur de matériau plus grande du bras de support.

5. Noyau de semelle (100) selon la revendication 4, dans lequel une zone variable (315, 325) pour l'adaptation de la largeur de matériau est prévue au niveau du bras de support.

6. Noyau de semelle (100) selon l'une quelconque des revendications précédentes qui est formé en plusieurs parties à partir d'au moins un support (200) et au moins un croisillon de soutien (300).

7. Noyau de semelle (100) selon l'une quelconque des revendications précédentes, dans lequel le support (200) et le croisillon de soutien (300) ensemble sont formés de manière intégrale.

8. Semelle de chaussure orthopédique, comprenant le noyau de semelle (100) selon l'une quelconque des revendications précédentes.
